Europäisches Patentamt

(19) European Patent Office

Office européen des brevets

(11) Publication number : **0 605 433 B1**

(12) **EUROPEAN PATENT SPECIFICATION**

(45) Date of publication of patent specification :
27.12.95 Bulletin 95/52

(51) Int. Cl.[6] : **G01N 33/569,** C07K 14/15

(21) Application number : 92915681.8

(22) Date of filing : 24.07.92

(86) International application number :
PCT/CA92/00322

(87) International publication number :
WO 93/03376 18.02.93 Gazette 93/05

(54) IMMUNOASSAY FOR THE SIMULTANEOUS DETECTION OF HIV-1, HIV-2, HTLV-I AND HTLV-II ANTIBODIES

(30) Priority : 29.07.91 US 737072

(43) Date of publication of application :
13.07.94 Bulletin 94/28

(45) Publication of the grant of the patent :
27.12.95 Bulletin 95/52

(84) Designated Contracting States :
AT CH DE ES FR GB IT LI

(56) References cited :
EP-A- 0 136 798
EP-A- 0 326 490
WO-A-89/01527
WO-A-90/08162

(72) Inventor : LACROIX, Martial Biochem Pharma
Inc.
2550 Daniel Johnson Blvd
Suite 600
Laval, Quebec H7T 2L1 2L1 (CA)
Inventor : DWYER, Robert, J. Biochem Pharma
Inc.
2550 Daniel Johnson Boulevard
Suite 600
Laval, Quebec H7T 2L1 (CA)
Inventor : ZREIN, Maan Biochem Pharma Inc.
2550 Daniel Johnson Boulevard
Suite 600
Laval, Quebec H7T 2L1 (CA)

(74) Representative : Gillard, Marie-Louise et al
Cabinet Beau de Loménie
158, rue de l'Université
F-75340 Paris Cédex 07 (FR)

(73) Proprietor : BIOCHEM IMMUNOSYSTEMS INC.
10900 Hamon Street
Montreal Quebec H3M 3A2 (CA)

EP 0 605 433 B1

## Description

TECHNICAL FIELD OF THE INVENTION

This invention relates to immunoassay kits which can be used to detect simultaneously antibodies in a biological specimen to the following viruses: HIV-1, HIV-2, HTLV-I and HTLV-II. This invention also relates to a process of making these immunoassay kits.

BACKGROUND OF THE INVENTION

Human T-cell leukemia virus subtype I (HTLV-I) is a retrovirus closely associated with adult T-cell leukemia/lymphoma (ATL) (Poiesz et al., **1980**, Proc. Natl. Acad. Sci. U.S.A., 77, 7415-7419). It is also linked with neurological diseases designated as tropical spastic paraparesis and HTLV-I-associated myelopathy (HAM) (Gessain et al., **1985**, Lancet ii, 407-410). Human T-cell leukemia virus subtype II (HTLV-II) was first isolated from a patient with T-cell hairy cell leukemia (Kalyanaraman et al., **1982**, Science, 218, 571-573). It is associated with a T-cell variant of hairy cell leukemia.

Adult T-cell leukemia/lymphoma (ATL) occurs mainly in Southwestern Japan, the Caribbean basin and parts of Central and South America. In those regions, seroprevalence varies between 5% and 15% in the general population and reaches 30% in older age groups. In the United States, HTLV-I/II infections are mainly present in intravenous drug users. In a recent American Red Cross survey (Williams et al., **1988,** Science, 240, 643-646), antibodies to HTLV-I could be detected in 10 of 39,898 random blood donors in eight U.S. cities; this represents a seroprevalence rate of 0.025%. A similar study involving 3158 individuals from Northern Egypt led to the identification of two carriers (prevalence rate of 0.06%) (El-Farrash et al., **1988**, Microbiol. Immunol., 32, 981-984). In these two studies, distinction between infection with HTLV I and HTLV-II was not clearly established.

Acquired Immune Deficiency Syndrome (AIDS), AIDS related complex (ARC) and pre-AIDS are also thought to be caused by a retrovirus, specifically the Human Immunodeficiency Virus (HIV). The first AIDS related virus, HIV-1 (also known as HTLV-III, LAV-1 and ARV) has been well characterized. Another pathogenic human retrovirus named HIV-2 (formerly LAV-2) has now been isolated from West African patients with AIDS. See, e.g., WO 87/04459. HIV-2 has recently been shown (Guyader et al., **1987**, Nature, 326, 662-669) to share a number of conserved sequences with HIV-1 and the Simian Immunodeficiency Viruses (SIV).

HTLV-I and HTLV-II both differ from the human immunodeficiency viruses HIV-1 and HIV-2 in their morphologic and genetic structures. As a result, antibodies to HIV antigens should not cross-react with HTLV-I and HTLV-II antigens. However, serum samples taken from patients with AIDS have shown some degree of cross-reactivity with HTLV antigens (Essex et al., **1983**, Science, 220, 859-862).

There are several examples in the art of diagnostic assays designed to detect independently antibodies to either HTLV antigens or HIV antigens. For example, Saxinger et al., **1984**, Science, 225, 1473-1476 refers to the use of a HTLV-I antigen as the immunoadsorbent in an enzyme immunoassay (EIA) for the detection of HTLV-I antibodies. Another example is Gnann et al., **1987**, J. Virol. 61, 2639-41, which refers to peptide sequences said to be useful in detecting HIV-1 antibodies.

Recently, there have been attempts in the art to provide diagnostic kits capable of simultaneously detecting antibodies to HTLV and HIV antigens. For example, Canadian patent 1,245,982 and European application 136,798 refer to assay kits for the simultaneous detection of antibodies to HTLV-I, HTLV-II and HTLV-III (HIV-1). In those kits the HTLV-antigens were derived from HTLV viral particles, obtained from various transformed cell lines. PCT application WO 90/08162, on the other hand, refers to an immuno assay kit to detect simultaneously antibodies to HTLV-1, HIV-1 and HIV-2 using antigens derived from the envelope protein of HTLV-1 and synthetic HIV peptides.

None of the above-described assays, however, provides a sensitive method to detect simultaneously antibodies to HTLV-I, HTLV-II, HIV-1 and HIV-2. In fact, even if the independent detection of antibodies to HIV-1, HIV-2, HTLV-I and HTLV-II is separately considered, none of the above-described assays provides a fully acceptable diagnostic test.

These prior assays are disadvantaged in various ways. In some cases, the tests utilize whole virus or bacterially produced immunoadsorbents. The use of these preparations is plagued by a lack of specificity -- i.e., false-positives -- because the blood of many non-infected individuals normally contains antibodies to contaminants commonly found in the preparations. False-positives also occur as a result of shared epitopes with viruses, which are unrelated to either HIV or HTLV and which may be present in these preparations. Another problem with these prior assays is lack of sensitivity. Lack of sensitivity allows contaminated blood to escape detection and thereby potentially infect blood product receivers and allow continued infectivity by undiagnosed HIV or HTLV carriers. The lack of sensitivity in these prior combination assays is thought to be due to the low surface density of epitopes on the solid phase of the assay when using whole virus or bacterially produced preparations. For these and other reasons, there still remains a great need for an assay that will simultaneously detect antibodies to HIV-1, HIV-2, HTLV-I and HTLV-II. Such a test would better insure that infected blood products do not enter blood banks and that infected individuals seek prompt and early treatment.

SUMMARY OF THE INVENTION

The assay kits according to the present invention solve the problems outlined above by Providing a sensitive and specific means and method of simultaneously detecting antibodies to HIV-1, HIV-2, HTLV-I and HTLV-II in samples of body fluids.

The assay kits of this invention comprise peptides, useful for the simultaneous detection of antibodies against HTLV-I, HTLV-II, HIV-1 and HIV-2, coated on a solid support.

The present invention further provides processes for making and using assay kits for the simultaneous detection of antibodies against HTLV-I HTLV-II, HIV-1 and HIV-2.

The assay kits of the present invention comprise at least four different HIV and HTLV peptides, two for HIV and two for HTLV. At least one of the HIV peptides is capable of recognizing an antibody to HIV-1 and at least one of the other HIV peptides is capable of recognizing an antibody to HIV-2. Similarly, at least one of the HTLV peptides is capable of recognizing an antibody to HTLV-I and at least one of the other HTLV peptides is capable of recognizing an antibody to HTLV-II.

Surprisingly, it has been found that when the HTLV-I and II peptides, and more preferably a cocktail containing them, is added first to the solid support, and then the HIV peptides added, separately or more preferably in a cocktail, the sensitivity of the resulting assay is greatly improved. Therefore, it is also an object of this invention to provide a method for making an assay kit for simultaneously detecting antibodies to HIV-1, HIV-2, HTLV-I and HTLV-II, wherein the HTLV-I and II peptides, preferably in the form of a cocktail, are first added to the support and then the HIV-1 and HIV-2 peptides, again preferably as a cocktail, are added to the support.

The kits of this invention may be used to screen a wide variety of biological specimens and fluids. These include blood, plasma, serum, saliva, urine, tears, milk or cerebrospinal fluid.

The HIV-1 peptides useful in the kits and methods of this invention are selected from the group of peptides of the formula, a-X-b, wherein X is selected from the group consisting of:

Arg Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser                    (BCH-87c)


Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser                    (BCH-87ck)


Lys Ile Glu Pro Leu Gly Val Ala Pro Thr Lys Ala Lys Arg

Arg Val Val Gln Arg Glu Lys Arg                 (BCH-132)


Lys Lys Ile Leu Ala Val Glu Arg Tyr Leu Lys Asp Gln Gln

Leu Leu Gly Ile Trp Gly Cys Ser Gly Lys Leu Ile Cys Thr

Thr Ala Val Pro Trp Asn Ala Ser                 (BCH-266)

```
Lys Ile Leu Ala Val Glu Arg Tyr Leu Lys Asp Gln Gln Leu
                     ┌─────────────────────────┐
Leu Gly Ile Trp Gly Cys Ser Gly Lys Leu Ile Cys Thr Thr

Ala Val Pro Trp Asn Ala Ser Gly Lys Leu Ile     (BCH-408)
```

```
Lys Lys Ile Leu Ala Val Glu Arg Tyr Leu Lys Asp Gln Gln
                         ┌─────────────────────────┐
Leu Leu Gly Ile Trp Gly Cys Ser Gly Lys Leu Ile Cys Thr

Thr Ala Val Pro Trp Asn Ala Ser Gly Lys Leu Ile

                                          (BCH-408k)
```

and analogs thereof;

a is an amino terminus, one to eight amino acids, but preferably one to five amino acids and more preferably one to three amino acids, or a substituent effective to facilitate coupling or to improve the immunogenic or antigenic activity of the peptide; and

b is a carboxy terminus, one to eight amino acids, but preferably one to five amino acids and more preferably one to three amino acids, or a substituent effective to facilitate coupling or to improve the immunogenic or antigenic activity of the peptide.

The HIV-2 peptides useful in the kits and methods of this invention are selected from the group of peptides of the formula, a-Y-b, wherein Y is selected from the group consisting of:

```
Arg Val Thr Ala Ile Glu Lys Tyr Leu Gln Asp Gln Ala Arg
                     ┌─────────────────────────┐
Leu Asn Ser Trp Gly Cys Ala Phe Arg Gln Val Cys His Thr

Thr Val Pro Trp Val Asn Asp Ser              (BCH-202c)
```

```
Lys Val Thr Ala Ile Glu Lys Tyr Leu Gln Asp Gln Ala Arg
                     ┌─────────────────────────┐
Leu Asn Ser Trp Gly Cys Ala Phe Arg Gln Val Cys His Thr

Thr Val Pro Trp Val Asn Asp Ser              (BCH-202ck)
```

```
Lys Lys Val Thr Ala Ile Glu Lys Tyr Leu Gln Asp Gln Ala
                         ┌─────────────────────────┐
Arg Leu Asn Ser Trp Gly Cys Ala Phe Arg Gln Val Cys His

Thr Thr Val Pro Trp Val Asn Asp Ser          (BCH-265)
```

```
Lys Val Thr Ala Ile Glu Lys Tyr Leu Gln Asp Gln Ala Arg
                        ┌──────────────────────┐
Leu Asn Ser Trp Gly Cys Ala Phe Arg Gln Val Cys His Thr

Thr Val Pro Trp Val Asn Asp Ser Ala Phe Arg Gln Val

                                            (BCH-417)
```

```
Lys Val Thr Ala Ile Glu Lys Tyr Leu Gln Asp Gln Ala Arg
                        ┌──────────────────────┐
Leu Asn Ser Trp Gly Cys Ala Phe Arg Gln Val Cys His Thr

Thr Val Pro Trp Val Asn Asp Ser Gly Lys His Ile

                                            (BCH-422)
```

and analogs thereof; and a and b are as defined above.

The HTLV-I peptides useful in the kits and methods of this invention are selected from the group consisting of peptides of the formula, a-Z-b, wherein Z is selected from the group consisting of:

```
Pro His Ser Asn Leu Asp His Ile Leu Glu Pro Ser Ile Pro

Trp Lys Ser Lys Pro Tyr Val Glu Pro Thr Ala Pro Gln Val

Leu                                         (BCH-234)
```

```
Ala Ile Val Ser Ser Pro Ser His Asn Ser Leu Ile Leu Pro

Pro Phe Ser Leu Ser Pro Val Pro Thr Leu Gly Ser Arg

                                            (BCH-416)
```

and analogs thereof; and a and b are as defined above.

The HTLV-II peptides useful in the kits and methods of this invention are selected from the group consisting of peptides of the formula, a-W-b, wherein W is selected from the group consisting of:

```
Leu Val His Asp Ser Asp Leu Glu His Val Leu Thr Pro Ser

Thr Ser Trp Thr Thr Lys Ile Leu          (BCH-219)
```

```
Thr Ser Glu Pro Thr Gln Pro Pro Pro Thr Ser Pro Pro Leu

Leu Val His Asp Ser Asp Leu Glu His Val Leu   (BCH-456)
```

```
Thr Ser Glu Pro Thr Gln Pro Pro Pro Thr Ser Pro Pro Leu

Val His Asp Ser Asp Leu Glu His Val Leu       (BCH-486)
```

and analogs thereof; and a and b are as defined above.

At least one peptide from each group -- HIV-1, HIV-2, HTLV-I and HTLV-II -- is used in the assay kits and methods of this invention.

DETAILED DESCRIPTION OF THE INVENTION

The present invention provides immunoassay kits comprising at least four peptides which, respectively, contain epitopes that recognize and selectively bind antibodies to HIV-1, HIV-2, HTLV-I and HTLV-II.

As defined above, the individual peptides of these four groups have the formula: a-X-b, a-Y-b, a-Z-b and a-W-b, respectively. Preferably, one member of each group is used in the kits and methods of this invention. However, this invention is not so limited. For example, more than one peptide of at least one of the groups or several peptides from each group may also be used. All that is required is that at least one member of each group of peptides a-X-b, a-Y-b, a-Z-b and a-W-b is used.

As used herein "analogs" denote amino acid insertions, deletions, substitutions and modifications at one or more sites in the described peptide chain.

Preferred modifications and substitutions to the amino acid sequence of the peptides of this invention are conservative ones (i.e., those having minimal influence on the secondary structure and hydropathic nature of the peptide). These include substitutions such as those described by Dayhoff in the Atlas of Protein Sequence and Structure 5, 1978 and by Argos in EMBO J. 8, 779-785, 1989. For example, amino acids belonging to one of the following groups represent conservative changes: ala, pro, gly, glu, asp, gln, asn, ser, thr; cys, ser, tyr, thr; val, ile, leu, met, ala, phe; lys, arg, his; and phe, tyr, trp, his. In like manner, methionine, an amino acid which is prone to oxidation may be replaced by norleucine. The preferred substitutions also include substitutions of D-isomers for the corresponding L-amino acids.

The term "amino acid" as employed in this description (e.g., in the definition of a and b) encompasses all of the natural amino acids, those amino acids in their D-configurations, and non-native, synthetic, and modified amino acids, such as homocysteine, ornithine, norleucine and β-valine.

As set forth briefly above, it is often useful and certainly within the scope of this invention to modify the peptides of this invention in order to make the chosen peptide more useful as an immunodiagnostic reagent or as an active ingredient of a vaccine. Such changes, for example, include:

- addition of a cysteine residue to one or both terminals in order to facilitate coupling of the peptide to a suitable carrier with heterobifunctional cross-linking reagents such as sulfosuccinimidyl-4-(p-maleimidophenyl) butyrate, preferred reagents for effecting such linkages are sulfosuccinimidyl-4-(N-maleimido-methyl)cyclohexane-1-carboxylate and N-succinimidyl-3-(2-pyridyldithio) propionate;

- addition of 1 to 8 additional amino acids (perferably 1 to 5, and most preferably 1 to 3 amino acids) at one or both terminals of the peptide to facilitate linking of the peptides to each other, for coupling to a support or larger peptide or protein or for modifying the physical or chemical properties of the peptide. Examples of such changes are the addition of N- or C-terminal tyrosine, glutamic acid or aspartic acid as linkers via an esterification reaction and lysine which can be linked via Schiff base or amide formation. As described above such additional amino acids may include any of the natural amino acids, those amino acids in their D-configurations, and the known non-native, synthetic and modified amino acids; and

- derivatization of one or both terminals of the peptide by, for example, acylation or amidation. These modifications result in changes in the net charge on the peptide and can also facilitate covalent linking of the peptide to a solid support, a carrier or another peptide. Examples of the substituents effective to facilitate coupling or to improve the immunogenicity or antigenic activity of the peptide are $C_2$-$C_{16}$ acyl groups, polyethylene glycol and phospholipids.

To prepare the peptides of this invention any of the conventional peptide production methodologies may be used. These include synthesis, recombinant DNA technology and combinations thereof. We prefer solid phase synthesis. In that synthetic approach, the resin support may be any suitable resin conventionally employed in the art for the solid phase preparation of peptides. Preferably, it is a p-benzyloxyalcohol polystyrene or p-methylbenzydrylamine resin. Following the coupling of the first protected amino acid to the resin support, the amino protecting group is removed by standard methods conventionally employed in the art. After removal of the amino protecting group, the remaining protected amino acids and, if necessary, side chain protected amino acids are coupled, sequentially, in the desired order to obtain the chosen peptide. Alternatively, multiple amino acid groups may be coupled using solution methodology prior to coupling with the resin-supported amino acid sequence.

The selection of an appropriate coupling reagent follows established art. For instance, suitable coupling reagents are N,N′-diisopropylcarbodiimide or N,N′-dicyclohexylcarbodiimide (DCC) or benzotriazol-1-yloxy-tris(dimethylamino)phosphonium hexafluoro-phosphate either alone or preferably in the presence of 1-hydroxybenzotriazole. Another useful coupling procedure employs preformed symmetrical anhydrides of protected amino acids.

The necessary α-amino Protecting group employed for each amino acid introduced onto the growing Polypeptide chain is preferably 9-fluorenylmethyloxycarbonyl (FMOC), although any other suitable protecting group may be employed as long as it does not degrade under the coupling conditions and is readily removable selectively in the presence of any other protecting group already present in the growing peptide chain.

The criteria for selecting protecting groups for the side chain amino acids are: (a) stability of the protecting group to the various reagents under reaction conditions selective for the removal of the $\alpha$-amino protecting group at each step of the synthesis; (b) retention of the protecting group's strategic properties (i.e., not be split off under coupling conditions) and (c) removability of protecting group easily upon conclusion of the peptide synthesis and under conditions that do not otherwise affect the peptide structure.

The fully protected resin-supported peptides of this invention are preferably cleaved from the p-benzyloxy alcohol resin with a 50% to 60% solution of trifluoroacetic acid in methylene chloride for 1 to 6 hours at room temperature in the presence of appropriate scavengers such as anisole, thioanisole, ethyl methyl sulfide, 1,2-ethanedithiol and related reagents. Simultaneously, most acid labile side-chain protecting groups are removed. More acid resistant protecting groups are typically removed by HF treatment.

The peptides of the present invention are useful as diagnostic reagents for the simultaneous detection of HIV-1, HIV-2, HTLV-I or HTLV-II-associated antibodies in accordance with methods well-known in the art. These include ELISA, hemagglutination, single-dot and multi-dot methods and assays.

Preferably, the test kits of this invention comprise an enzyme-linked immunosorbent assay (ELISA). In this preferred embodiment, a peptide or mixture of several peptides of this invention is adsorbed onto, or covalently coupled to a solid support like, for example, the wells of a microtiter plate. Surprisingly, when the HTLV-I/II peptides (or preferably a mixture of them) is added first to the wells, i.e., before the HIV1/2 peptides are added to the wells, the sensitivity of the assay is greatly enhanced. Therefore, the preferred embodiment of this invention comprises a kit, and a method for making such a kit, wherein a mixture or cocktail of the HTLV-I and II peptides is first coupled to the support and then the HIV-1 and HIV-2 peptides, and more preferably a mixture of them, is coupled to the support.

Suitable solid supports for the assays and methods of this invention include organic and inorganic polymers, e.g., amylases, dextrans, natural or modified celluloses, polyethylene, Polystyrene, polyacrylamides, agaroses, magnetite, porous glass powder, polyvinyldiene fluoride (kynar) and latex, the inner wall of test vessels (i.e., test tubes, titer plates or cuvettes of glass or artificial material) as well as the surface of solid bodies (i.e., rods of glass and artificial material, rods with terminal thickening, multi-well rods with terminal lobes or lamellae). Multi-well plastic microtiter plates are especially suitable, and preferred, as solid phase carriers.

The peptides and mixtures thereof used in this invention may be applied to the solid support by any method and in any amount effective to maximize the sensitivity and specificity of the resulting kit. We prefer to contact the solid support with a solution comprising one or more peptides of this invention dissolved in a buffer. Although the buffer may be any buffer conventionally used to dissolve peptides and apply them to solid supports, we prefer to use a 0.05 M sodium carbonate-bicarbonate solution (pH 9.6).

The concentration of peptide in the buffer should be sufficient to allow an effective amount of peptide to adhere to the surface of the solid support. As used herein, the term "effective amount" refers to an amount of peptide that on a solid support effectively detects antibodies to HIV-1, HIV-2, HTLV-I or HTLV-II, respectively, in a biological specimen.

Preferably, the peptide solution used to coat the wells of a microtiter plate in accordance with this invention has a peptide concentration of between 5 $\mu$g/ml to 10 $\mu$g/ml, and more preferably 10 $\mu$g/ml. Such a peptide concentration may be.due to a single peptide if only a single peptide is present in the solution or it may be due to the total concentration of all the peptides in a cocktail. The peptide cocktails of this invention are typically and preferably prepared by mixing solutions containing 5 $\mu$g/ml to 10 $\mu$g/ml of the individual peptides of this invention. For example, if a cocktail of two peptides is to be prepared, a chosen volume of 5 $\mu$g/ml to 10 $\mu$g/ml solution of peptide 1 would be mixed with a chosen volume of a 5 $\mu$g/ml to 10 $\mu$g/ml solution of peptide 2. The resulting cocktail would as a result have a total peptide concentration of 5 $\mu$g/ml to 10 $\mu$g/ml, and preferably 10 $\mu$g/ml.

The particular volumes chosen to make the peptide cocktails of this invention are determined somewhat empirically by testing the resultant assay kit for sensitivity and specificity in the detection of antibodies to HIV-1, HIV-2, HTLV-I and HTLV-II. The actual volumes of the peptide solutions mixed to form the peptide cocktail may then be independently varied within the parameters set forth above to achieve maximum sensitivity and maximum specificity in the resultant assay kit, it being understood that the peptide cocktails need not contain equal amounts of each component peptide. In fact, we prefer to use peptide cocktails that contain more of each of BCH-408 and BCH-202 ck, than BCH 132 and more BCH 456 than each of BCH 234 and BCH 416.

In a preferred embodiment of this invention, we combine the HTLV-I and HTLV-II peptide solutions (5 $\mu$g/ml to 10 $\mu$g/ml, and preferably 10 $\mu$g/ml) to form an HTLV peptide cocktail before applying those peptides to the solid support. In like manner, we prefer to combine the HIV-1 and HIV-2 peptide solutions (5 $\mu$g/ml to 10 $\mu$g/ml, and preferably 10 $\mu$g/ml) to form an HIV peptide cocktail before applying them to the solid support. The preferred peptide compositions useful in the kits of this invention are: (1) an HIV-1/2 peptide cocktail containing BCH-132, BCH-202 ck and BCH-408 in a ratio of 1:40:60 and (2) an HTLV-I/II peptide cocktail comprising BCH-234, BCH-416 and BCH-456 in a ratio of 1:1:4. The ratios and concentrations of peptides in other assay kits of this invention may, of course, be varied to maximize the sensitivity and specificity of the resulting kits.

The peptide solutions are contacted with the wells in accordance with this invention using conventional techniques. In the embodiment where the well is coated separately with each individual peptide, we typically use 60 µl of the first peptide solution; 120 µl of the second, 180 µl of the third and 240 µl of the fourth. This allows different surfaces of the well to be coated with each peptide. In the preferred embodiment where we use an HTLV cocktail and an HIV cocktail, we typically use 120 µl of the HTLV cocktail and 240 µl of the HIV cocktail -- again for the same reason.

The amount of time in which the peptide solution or peptide cocktail is in contact with the solid support should be sufficient to allow an effective amount of the peptide or mixture of peptides to adhere to the surface of the support material. Preferably, 1 hour at room temperature is enough. However, at 4°C we usually contact the peptides and well overnight. After such a time, we prefer to remove any uncoupled peptide. This step may be carried out using any of the conventional methods for removing excess liquid from solid supports. We prefer to use aspiration. After the aspiration, the well may also be washed with a washing buffer such as a sodium phosphate buffer (NaCl, 0.15 M; $NaH_2PO_4$; 0.06 M; thimerosal, 0.01% and Tween 20, 0.05%; pH 7.4). However, this washing step is not required. After aspiration and/or washing, additional peptides or mixtures thereof may then be applied to the solid support by repeating the application procedure set forth above.

In another embodiment of this invention, we do not remove the excess of the first peptide or cocktail from the well before contacting the well with the next peptide or cocktail. In this embodiment, we prefer to use approximately equal volumes of each peptide or cocktail. For example, if single peptide solutions are used separately, we would use 60 µl of the first peptide and after 1 hour, add 60 µl of the second peptide ans so on. If the HTLV and HIV peptide cocktails were being used, we would use 120 µl of the HTLV cocktail and after 1 hour add 120 µl of the HIV cocktail. After an additional hour, the excess solution would be removed and the well usually washed with buffer.

After the desired peptides are coupled to the support material and any excess materials removed, the wells are treated with the biological specimen to be tested. Preferably, this specimen is serum although other specimens such as blood, plasma, saliva, urine, tears, milk or cerebrospinal fluid may also be used. The biological specimen may also be diluted with a specimen buffer before assay. An example of such a buffer is a sodium phosphate buffer (sodium phosphate, 6 mM; NaCl, 0.15 M; BSA, 2%; peptone, 1.5%; benzamidine, 80 mM; Tween 20, 1%; heat-inactivated goat serum, 40%; thimerosal 0.01%, final pH 7.2). The volume of the specimen solution added per well is preferably equal to five sixths of the maximum volume used to coat the well. For example, if 240 µl of peptide solution were used to coat the well then the preferred volume of specimen solution to be added would be 200 µl.

After exposure to the sample to be analyzed, the wells are emptied, preferably using aspiration, and washed. After washing, anti-human IgG or anti-human IgM labeled with peroxidase is added to the wells. The determination of the peroxidase is typically performed with a corresponding substrate, e.g., 3,3′,5,5′-tetramethylbenzidine. Without departing from the usefulness of this illustrative assay, the peroxidase can be exchanged by another label, e.g., by radioactive, fluorescence, chemiluminescence or infra-red emitting or absorbing labels.

General procedures for the synthesis and utilization of the peptides of this invention are provided below.

Procedure 1: Preparation of Resins Carrying the N-FMOC Protected Amino Acid Residue

The desired N-FMOC protected amino acid residue in a mixture of methylene chloride ($CH_2Cl_2$) and dimethylformamide (DMF) (4:1) was added to a suspension of p-benzyloxy alcohol resin in $CH_2Cl_2$:DMF (4:1) at 0°C. The mixture was stirred manually for a few seconds and then treated with N,N′-dicyclohexyl-carbodiimide (DCC) followed by a catalytic amount of 4-(dimethylamino) pyridine. The mixture was stirred at 0°C for an additional 30 minutes and then at room temperature overnight. The filtered resin was washed successively with $CH_2Cl_2$, DMF and isopropanol (3 washes each) and finally, with $CH_2Cl_2$. The resin was suspended in $CH_2Cl_2$, chilled in an ice bath and redistilled pyridine was added to the stirred suspension followed by benzoyl chloride. Stirring was continued at 0°C for 30 minutes and then at room temperature for 60 minutes. After filtration, the resin was washed successively with $CH_2Cl_2$, DMF and isopropanol (3 washes each) and finally with petroleum ether (twice) before being dried under high vacuum to a constant weight.

Spectrophotometric determination of substitution according to Meienhofer et al. (Int. J. Peptide Protein Res., 13, 35, 1979) indicates the degree of substitution on the resin.

Procedure 2: Coupling of Subsequent Amino Acids

The resin carrying the N-FMOC protected first amino acid residue was placed in a reaction vessel of a Biosearch 9600 Peptide Synthesizer and treated as follows:

1) Washed with DMF (4 times for 20 sec. each)
2) Prewashed with a 30% solution of piperidine in DMF (3 min.)
3) Deprotected with a 30% solution of piperidine in DMF (7 min.)
4) Washed with DMF (8 times for 20 sec. each)
5) Checked for free amino groups - Kaiser Test (must be positive)
6) The peptide resin was then gently shaken for 1 or 2 hrs with 8 equivalents of the desired FMOC-protected amino acid and 1-hydroxybenzotriazole and benzotriazol-1-yloxy-tris(dimethyl-amino)phosphonium hexafluorophosphate all dissolved in dry redistilled DMF containing 16 equivalents of 4-methylmorpholine.
7) Washed with DMF (6 times for 20 sec. each)

After step 7, an aliquot was taken for a ninhydrin test. If the test was negative, one goes back to step 1 for coupling of the next amino acid. If the test was positive or slightly positive, steps 6 and 7 should be repeated.

The above scheme may be used for coupling each of the amino acids of the peptides described in this invention. N-protection with FMOC may also be used with each of the remaining amino acids throughout the synthesis.

Radiolabeled peptides may be prepared by incorporation of a tritiated amino acid using the above coupling protocol.

After the addition of the last amino acid, the N-FMOC of the N-terminal residue is removed by going back to steps 1-7 of the above scheme. The peptide resin is washed with $CH_2Cl_2$ and dried in vacuo to give the crude protected peptide.

Procedure 3: Deprotection and Cleavage of the Peptides from the Resin

The protected peptide-resin was suspended in a 55% solution of trifluoroacetic acid (TFA) in $CH_2Cl_2$, containing 2.5% ethanedithiol and 2.5% anisole. The mixture was flushed with $N_2$ and stirred for 1.5 hours at room temperature. The mixture was filtered and the resin washed with $CH_2Cl_2$. The resin was treated again with 20% TFA in $CH_2Cl_2$ for 5 minutes at room temperature. The mixture was filtered and the resin washed with 20% TFA in $CH_2Cl_2$ and then washed with $CH_2Cl_2$. The combined filtrates were evaporated in vacuo below 35°C and the residue triturated several times with dry dimethyl ether. The solid was dissolved in 10% aqueous acetic acid and lyophilized to afford the crude product.

The peptides containing arg and cys residues are further deprotected by HF treatment at 0°C for 1 hour in the presence of anisole and dimethylsulfide. The peptides were extracted with 10% aqueous acetic acid, washed with dimethyl ether and lyophilized to afford the crude peptides.

Procedure 4: Purification of Peptides

The crude peptides were purified by preparative HPLC on a Vydac column (2.5 X 25 mm) of $C_{18}$ or $C_4$ reverse phase with a gradient of the mobile phase. The effluent was monitored at 220 nm and subsequently by analytical HPLC. Relevant fractions were pooled, evaporated and lyophilized. The identity of the synthetic peptides was verified by analytical reverse phase chromatography and by amino acid analysis.

Procedure 5: Simultaneous detection of antibodies to HIV-1, HIV-2, HTLV-I and HTLV-II by an Enzyme-Linked Immunosorbent Assay (ELISA)

The synthetic peptides were solubilized in 0.05 M sodium carbonate - bicarbonate buffer (pH 9.6) to form peptide solutions having peptide concentrations between about 5 µg/ml to about 10 µg/ml. Two peptide cocktails were then prepared by mixing volumes of these individual peptide solutions. The first cocktail included synthetic peptides for HTLV-I and HTLV-II. The second cocktail included synthetic peptides for HIV-1 and HIV-2. The total peptide concentration of each cocktail was between 5 µg/ml and 10 µg/ml.

The HTLV-I/II cocktail was added to the wells first. Then, the HIV-1/2 peptide cocktail was coupled to the support. In the examples that follow, 120 µl of the HTLV-I/II-derived synthetic peptide cocktail was first added to each well and the plates incubated overnight at 4°C. The wells were then emptied and filled with 240 µl of the HIV-1/2-derived synthetic peptide cocktail. The plates were incubated overnight at 4°C. We used an Oyster Bay Versafill dispensing system to fill the wells. The wells were emptied by aspiration and washed twice with a washing buffer (NaCl, 0.15 M; $NaH_2PO_4$, 0.06 M; thimerosal, 0.01% and Tween 20, 0.05%; pH 7.4 [0.3 ml-

/well]). The wells were then saturated with 0.35 ml of washing buffer for 1 hour at 37°C and washed once with the same buffer without Tween 20. The empty plates were dried for 1 hour at 37°C and vacuum sealed until used.

Serum samples to be analyzed were diluted with specimen buffer (sodium phosphate, 6 mM; NaCl, 0.15 M; BSA, 2%; peptone, 1.5%; benzamidine, 80 mM; Tween 20, 1%; heat-inactivated goat serum, 40%; thimerosal 0.01%, final pH 7.2) and diluted serum samples (0.2 ml) were added to the wells. The filled wells were left to incubate for 30 minutes at room temperature. The wells were then emptied and washed 5 times with washing buffer.

A conjugate solution (peroxidase labeled affinity purified goat antibody to human IgG, 0.5 mg in 5 ml 50% glycerol) diluted with 1% w/v bovine serum albumin in a solution containing Tris, 0.05 M; NaCl, 0.5 M; Tween 20, 0.05%; thimerosal 0.01% (pH 7.2) was added to each well (0.2 ml) and incubated for 30 minutes at room temperature. The wells were then emptied and washed five times with the washing buffer. A substrate solution (2 mg/ml 3,3′,5,5′-tetra-methylbenzidine in N-methyl pyrrolidone (NNP)) in acetate buffer (0.1 M; pH 5.6) was diluted with 5 volumes peroxide solution containing 0.015% v/v of 30% $H_2O_2$ and added to each well (0.2 ml per well). After 30 minutes, the contents of each well were treated with 0.1 ml 1N $H_2SO_4$ and the optical density was read at 450 nm. All determinations were done in duplicate.

Using procedures substantially as described above, the specific peptides discussed below were prepared and then evaluated for their ability to detect HIV-1, HIV-2, HTLV-I and HTLV-II-specific antibodies.

## Experiment 1

In this experiment, the HTLV-peptide cocktail had a total peptide concentration of 10 μg/ml and was composed of a mixture of BCH-219, BCH-234 and BCH-416 in a 1:1:1 ratio, each peptide first being solubilized in the carbonate buffer described in Procedure 5 to a concentration of 10 μg/ml. As described above, 120 μl of this HTLV-I/II synthetic peptide cocktail was added to each well and incubated overnight at 4°C. The wells were emptied and 240 μl of the HIV-1/2 peptide cocktail was added. This HIV-1/2 cocktail was formed by mixing 3 volumes of a BCH-408 peptide solution (10 μg/ml) with two volumes of a BCH-202ck solution (10 μg/ml) to yield a cocktail having a total peptide concentration of 10 μg/ml, containing BCH-408 and BCH-202ck in a 3:2 ratio. Adsorption of peptides present in this second cocktail and further processing of the plates was done as described in Procedure 5. Two series of plates were also prepared using 120 μl of each peptide cocktail (i.e., one series for HIV-1/2-derived synthetic peptides and one series for HTLV-I/II-derived synthetic peptides). These plates (HIV-EIA and HTLV-EIA) were used in side-by-side studies with the HTLV/HIV combined plates (combined HIV/HTLV-EIA) to compare their performance in detecting antibodies to HIV and HTLV in blood serum samples. Table 1 displays this comparison.

## TABLE 1

| Sample No. | Status | | Signal/cutoff ratios recorded using: | | |
|---|---|---|---|---|---|
| | | | HIV-EIA | HTLV-EIA | Combined HIV/HTLV-EIA |
| NC | Negative | | 0.22 | 0.11 | 0.29 |
| NC | Negative | | 0.24 | 0.37 | 0.31 |
| NC | Negative | | 0.24 | 0.20 | 0.34 |
| PC/HIV-1 | HIV-1 | pos | 11.11 | 0.25 | >12 |
| PC/HIV-2 | HIV-2 | pos | 10.45 | 0.24 | >12 |
| PC/HTLV-I | HTLV-I | pos | | 6.55 | 8.73 |
| PC/HTLV-II | HTLV-II | pos | | 6.82 | 8.98 |
| A02-1 | HTLV-II | pos | | 5.62 | 4.04 |
| A02-2 | HTLV-I | pos | | >12 | >12 |
| A02-3 | HTLV-II | pos | | 0.84 | 0.69 |
| A02-4 | HTLV-II/ HIV-1 | pos | | 2.95 | >12 |
| A02-5 | HTLV-II | pos | | 1.26 | 1.62 |
| A02-6 | Ind. | | | 0.18 | 0.45 |
| A02-7 | HTLV-I | pos | | 8.73 | 10.15 |
| A02-8 | HTLV-II | pos | | 1.71 | 1.56 |
| A02-9 | HTLV/II HIV-1 | pos | | 10.98 | >12 |
| A02-10 | HTLV-I | pos | | >12 | >12 |
| A02-11 | HTLV-II | pos | | 3.83 | 3.42 |

**TABLE 1 (cont.)**

| | | Signal/cutoff ratios recorded using: | | |
|---|---|---|---|---|
| Sample No. | Status | HIV-EIA | HTLV-EIA | Combined HIV/HTLV-EIA |
| A02-12 | HTLV-II pos | | 1.32 | 1.35 |
| A02-13 | Ind. | | 0.79 | 1.05 |
| A02-14 | HTLV-II pos | | 7.97 | 10.01 |
| A02-15 | HTLV-II pos | | 3.83 | 3.26 |
| A02-16 | HTLV-II pos | | 1.89 | 2.21 |
| A02-17 | Ind. | | 0.90 | 1.02 |
| A02-18 | HTLV-II pos | | 1.28 | 1.09 |
| A02-19 | HTLV-II pos | | 3.42 | 2.07 |
| A02-20 | HTLV-I pos | | >12 | >12 |
| A02-21 | HTLV-II pos | | 1.43 | 1.70 |
| A02-22 | HTLV-II pos | | 5.56 | 4.83 |
| A02-23 | HTLV-II pos | | 3.29 | 2.84 |
| A02-24 | HTLV-II pos | | 13.53 | 12.35 |
| A02-25 | HTLV-II pos | | 1.03 | 1.06 |
| C-20 | HIV-1 seroc. | 0.14 | | 0.29 |
| C-21 | HIV-1 seroc. | 0.24 | | 0.38 |
| C-22 | HIV-1 seroc. | 0.46 | | 0.60 |
| C-23 | HIV-1 seroc. | 2.91 | | 2.54 |
| C-24 | HIV-1 seroc. | 4.17 | | 3.58 |
| C-25 | HIV-1 Seroc. | 5.99 | | 4.89 |
| G-80 | HIV-1 seroc. | 0.18 | | 0.37 |
| G-81 | HIV-1 seroc. | 0.26 | | 0.30 |
| G-82 | HIV-1 seroc. | 0.30 | | 0.39 |
| G-83 | HIV-1 seroc. | 4.96 | | 4.03 |
| G-84 | HIV-1 seroc. | 5.79 | | 3.84 |
| G-85 | HIV-1 seroc. | 4.85 | | 3.67 |
| G-86 | HIV-1 seroc. | 5.89 | | 5.01 |

The results in Table 1 represent signal to cutoff ratios. The cutoff is the average absorbency obtained with plasma samples drawn from three normal blood donors to which a value of 0.10 is added. A ratio $\geq 1.0$ indicates a positive sample.

Samples labeled "NC" in Table 1 were negative controls obtained from a patient verified as HIV-1/2 negative and HTLV-I/II negative. Samples labeled "PC" were positive controls obtained from patients verified respectively as only HIV-1 positive, only HIV-2 positive, only HTLV-I positive and only HTLV-II positive. Samples labeled "A02" (1 to 25), "C" (20 to 25) and "G" (80 to 86) were obtained from Boston Biomedica Inc. (BBI). Their status was determined by BBI. "Ind." indicates an indeterminate Western Blot profile. "HIV-1 seroc." indicates that these plasma samples were sequentially collected from a donor who was retrospectively found to have been infected with HIV-1. These patients produced antibodies to HIV-1 several weeks after testing negative for these same antibodies. The samples are numbered chronologically.

In general, the signal to cut-off ratios measured with the HIV/HTLV combined plate are comparable to those measured with the plates coated separately with only HIV-peptides or with plates coated separately with only HTLV-peptides. This is a surprising result because a significant signal reduction would have been expected upon dilution of the epitope which recognizes a given antibody. In the case of co-infected HIV/HTLV samples, i.e., A02-4, the signal to cutoff ratio recorded with the HIV/HTLV-plate (>12) is significantly _higher_ than the one measured on the plate coated with only HTLV-peptides (2.95).

HIV-1 seroconversions of patients C and G from anti-HIV-1 negative to anti-HIV-1 positive are detected simultaneously on the HIV- plate and the combined HIV/HTLV- plate.

Although the combined HIV/HTLV- plate performs as well as the HTLV- peptide coated plate, in several cases (samples A02-3, A02-13, and A02-17) samples containing antibodies to HTLV-II were not detected as positive in either the combined HIV-HTLV or the single HTLV plates. Other cocktails were thus assembled and tested in an attempt to improve sensitivity towards HTLV-antibodies.

Experiment 2

This experiment is very similar to the preceeding one. The composition of the two peptide cocktails were as follows: the HTLV peptide cocktail contained a total peptide concentration of 10 µg/ml and comprised BCH-234, BCH-416 and BCH-456 in a 1:1:4 ratio. It was prepared using individual 10 µg/ml peptide solutions. The HIV-peptide cocktail contained a total peptide concentration of 5 µg/ml and comprised BCH-132, BCH-202ck and BCH-408 in a 1:40:60 ratio. It was prepared using individual 5 µg/ml peptide solutions. Table 2 displays the results obtained in wells coated with these peptides.

TABLE 2

| Sample No. | Status | Combined HIV/ HTLV-EIA | Signal/cutoff measured |
|---|---|---|---|
| NC | Negative | | 0.32 |
| NC | Negative | | 0.68 |
| NC | Negative | | 0.40 |
| PC/HIV-1 | HIV-1 | pos | >10 |
| PC/HIV-2 | HIV-2 | pos | >10 |
| PC/HTLV-I | HTLV-I | pos | 8.63 |
| PC/HTLV-II | HTLV-II | pos | 8.74 |
| A02-1 | HTLV-II | pos | >10 |
| A02-2 | HTLV-I | pos | >10 |
| A02-3 | HTLV-II | pos | 1.08 |
| A02-4 | HTLV/HIV | pos | >10 |
| A02-5 | HTLV-II | pos | 8.76 |
| A02-6 | Ind. | | 0.51 |
| A02-7 | HTLV-I | pos | 9.53 |
| A02-8 | HTLV-II | pos | 4.30 |
| A02-9 | HTLV/HIV | pos | >10 |
| A02-10 | HTLV-I | pos | >10 |
| A02-11 | HTLV-II | pos | 5.19 |
| A02-12 | HTLV-II | pos | 9.79 |
| A02-13 | Ind. | | 1.41 |
| A02-14 | HTLV-II | pos | 10.64 |
| A02-15 | HTLV-II | pos | 4.78 |
| A02-16 | HTLV-II | pos | 4.49 |
| A02-17 | Ind. | | 1.93 |
| A02-18 | HTLV-II | pos | 2.79 |
| A02-19 | HTLV-II | pos | >10 |
| A02-20 | HTLV-I | pos | >10 |
| A02-21 | HTLV-II | pos | 4.32 |
| A02-22 | HTLV-II | pos | 7.26 |
| A02-23 | HTLV-II | pos | 7.22 |
| A02-24 | HTLV-II | pos | >10 |
| A02-25 | HTLV-II | pos | 2.51 |

TABLE 2 (cont.)

| Sample No. | Status | Combined HIV/ HTLV-EIA | Signal/cutoff measured |
|---|---|---|---|
| C-20 | HIV-1 | seroc. | 0.32 |
| C-21 | HIV-I | seroc. | 0.39 |
| C-22 | HIV-1 | seroc. | 0.89 |
| C-23 | HIV-1 | seroc. | 2.29 |
| C-24 | HIV-1 | seroc. | 3.60 |
| C-25 | HIV-1 | seroc. | 5.36 |
| C-26 | HIV-1 | seroc. | 5.33 |
| C-27 | HIV-1 | seroc. | 8.41 |
| C-28 | HIV-1 | seroc. | 9.78 |
| C-29 | HIV-1 | seroc. | >10 |
| C-30 | HIV-1 | seroc. | >10 |
| C-31 | HIV-1 | seroc. | >10 |
| C-32 | HIV-1 | seroc. | >10 |
| C-33 | HIV-1 | seroc. | >10 |
| C-34 | HIV-1 | seroc. | >10 |
| C-35 | HIV-1 | seroc. | >10 |
| C-36 | HIV-1 | seroc. | >10 |
| C-37 | HIV-1 | seroc. | >10 |
| G-80 | HIV-1 | seroc. | 0.43 |
| G-81 | HIV-1 | seroc. | 0.51 |
| G-82 | HIV-1 | seroc. | 0.50 |
| G-83 | HIV-1 | seroc. | 4.35 |
| G-84 | HIV-1 | seroc. | 5.15 |
| G-85 | HIV-1 | seroc. | 4.39 |
| G-86 | HIV-1 | seroc. | 6.15 |
| G-87 | HIV-1 | seroc. | >10 |
| G-88 | HIV-1 | seroc. | >10 |
| G-89 | HIV-1 | seroc. | >10 |
| G-90 | HIV-1 | seroc. | >10 |
| G-91 | HIV-1 | seroc. | >10 |

The results presented in Table 2 show that the new peptide compositions greatly improved the sensitivity towards the HTLV-specific antibodies. For example, sample A02-3 went from 0.69 (Table 1) to 1.08 with the new peptide mixture. The eight samples that were low positive (1.02 to 1.70) with the cocktails used in Experiment 1 gave strong positive signal to cutoff ratios (1.93 to 9.79) with the peptide cocktails described in Experiment 2.

Sensitivity towards HIV- antibody detection was also improved but to a lesser extent. Most noteworthy is the increase in signal to cutoff ratio of sample C-22 0.60 to 0.89. Such an increase classifies this sample as "borderline negative." In many laboratories, borderline negative samples qualify for further testing and would thus have a greater chance of being detected as positive.

Another series of tests was done using plates having the same peptide ratios and made as described in Experiment 2 in order to evaluate further their sensitivity and specificity. Table 3a and Table 3b below summarize the data obtained.

Table 3a

| Status of samples tested | | No. tested | No. reactive | No. confirmed reactive |
|---|---|---|---|---|
| Anti-HIV-1 | positive | 45 | 45 | 45 |
| Anti-HIV-2 | positive | 19 | 19 | 19 |
| Anti-HIV-1 | (low titer panel) | 14 | 14 | 14 |
| Anti-HTLV-I | positive | 44 | 44 | 44 |
| Anti-HTLV-II | positive | 12 | 12 | 12 |

Table 3b

| Status of samples tested | No. tested | No. reactive | No. confirmed reactive | % specificity |
|---|---|---|---|---|
| Samples from pregnant Women | 432 | 2 | 1 | 99.8% |
| Routine HIV negative | 37 | 0 | 0 | 100% |
| Routine HTLV negatives | 34 | 2 | 2* | 100% |
| Normal blood donors | 792 | 2 | ND | $\geq$99.7% |

**\* Both samples were confirmed to be anti-HIV-1 positive**

These results show the impressive sensitivity demonstrated by this assay kit. In Table 3a, every one of the samples containing antibodies to HIV-1, HIV-2, HTLV-I and HTLV-II was properly detected as positive by the combined HIV/HTLV test kit. These samples included a low titer panel of serum samples that contain very low concentrations of antibodies to HIV-1.

Table 3b exemplifies the extraordinary specificity demonstrated by this assay kit. Of the 1295 samples screened, only 1 false-positive was confirmed. This degree of specificity is significantly better than the acceptable range of around 99.5%.

While we have herein before presented a number of embodiments of this invention, it is apparent that our basic construction can be altered to provide other embodiments which utilize the processes and compositions of this invention. Therefore, it will be appreciated that the scope of this invention is defined by the claims appended hereto rather than by the specific embodiments which have been presented hereinabove by way of example.

**Claims**

1. An immunoassay kit for simultaneously detecting antibodies to HIV-1, HIV-2, HTLV-I and HTLV-II comprising at least one peptide from each of four groups of peptides, coupled to a solid support, the peptides groups having the formulae a-X-b, a-Y-b, a-Z-b and a-W-b, wherein:

   X is selected from the group consisting of

```
Arg Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser                    (BCH-87c)


Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser                    (BCH-87ck)


Lys Ile Glu Pro Leu Gly Val Ala Pro Thr Lys Ala Lys Arg

Arg Val Val Gln Arg Glu Lys Arg                (BCH-132)


Lys Lys Ile Leu Ala Val Glu Arg Tyr Leu Lys Asp Gln Gln

Leu Leu Gly Ile Trp Gly Cys Ser Gly Lys Leu Ile Cys Thr

Thr Ala Val Pro Trp Asn Ala Ser               (BCH-266)


Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser Gly Lys Leu Ile    (BCH-408)

Lys Lys Ile Leu Ala Val Glu Arg Tyr Leu Lys Asp Gln Gln


Leu Leu Gly Ile Trp Gly Cys Ser Gly Lys Leu Ile Cys Thr

Thr Ala Val Pro Trp Asn Ala Ser Gly Lys Leu Ile

                                              (BCH-408k)
```

and analogs thereof;

   Y is selected from the group consisting of

```
Arg Val Thr Ala Ile Glu Lys Tyr Leu Gln Asp Gln Ala Arg
        ┌─────────────────────┐
Leu Asn Ser Trp Gly Cys Ala Phe Arg Gln Val Cys His Thr
Thr Val Pro Trp Val Asn Asp Ser              (BCH-202c)


Lys Val Thr Ala Ile Glu Lys Tyr Leu Gln Asp Gln Ala Arg
        ┌─────────────────────┐
Leu Asn Ser Trp Gly Cys Ala Phe Arg Gln Val Cys His Thr
Thr Val Pro Trp Val Asn Asp Ser              (BCH-202ck)


Lys Lys Val Thr Ala Ile Glu Lys Tyr Leu Gln Asp Gln Ala
            ┌─────────────────────┐
Arg Leu Asn Ser Trp Gly Cys Ala Phe Arg Gln Val Cys His
Thr Thr Val Pro Trp Val Asn Asp Ser          (BCH-265)


 Lys Val Thr Ala Ile Glu Lys Tyr Leu Gln Asp Gln Ala Arg
        ┌─────────────────────┐
Leu Asn Ser Trp Gly Cys Ala Phe Arg Gln Val Cys His Thr
Thr Val Pro Trp Val Asn Asp Ser Ala Phe Arg Gln Val
                                             (BCH-417)



Lys Val Thr Ala Ile Glu Lys Tyr Leu Gln Asp Gln Ala Arg
        ┌─────────────────────┐
Leu Asn Ser Trp Gly Cys Ala Phe Arg Gln Val Cys His Thr
Thr Val Pro Trp Val Asn Asp Ser Gly Lys His Ile
                                             (BCH-422)
```

and analogs thereof;
Z is selected from the group consisting of

```
Pro His Ser Asn Leu Asp His Ile Leu Glu Pro Ser Ile Pro
Trp Lys Ser Lys Pro Tyr Val Glu Pro Thr Ala Pro Gln Val
Leu                                          (BCH-234)


Ala Ile Val Ser Ser Pro Ser His Asn Ser Leu Ile Leu Pro
Pro Phe Ser Leu Ser Pro Val Pro Thr Leu Gly Ser Arg
                                             (BCH-416)
```

and analogs thereof;
W is selected from the group consisting of

17

```
Leu Val His Asp Ser Asp Leu Glu His Val Leu Thr Pro Ser

Thr Ser Trp Thr Thr Lys Ile Leu               (BCH-219)
```

```
Thr Ser Glu Pro Thr Gln Pro Pro Pro Thr Ser Pro Pro Leu

Leu Val His Asp Ser Asp Leu Glu His Val Leu    (BCH-456)
```

```
Thr Ser Glu Pro Thr Gln Pro Pro Pro Thr Ser Pro Pro Leu

Val His Asp Ser Asp Leu Glu His Val Leu        (BCH-486)
```

and analogs thereof;

     a is an amino terminus, one to eight amino acids or a substituent effective to facilitate coupling or to improve the immunogenic or antigenic activity of the peptide; and

     b is a carboxy terminus, one to eight amino acids or a substituent effective to facilitate coupling or to improve the immunogenic or antigenic activity of the peptide;

2.    The kit of claim 1, wherein peptides are made by solid phase synthesis.

3.    The kit of claim 1, wherein the HIV-1 peptides coupled to the solid support are:

```
Lys Ile Glu Pro Leu Gly Val Ala Pro Thr Lys Ala Lys Arg

Arg Val Val Gln Arg Glu Lys Arg            (BCH-132);
```

and

```
Lys Ile Leu Ala Val Glu Arg Tyr Leu Lys Asp Gln Gln Leu
                      ┌──────────────────────┐
Leu Gly Ile Trp Gly Cys Ser Gly Lys Leu Ile Cys Thr Thr

Ala Val Pro Trp Asn Ala Ser Gly Lys Leu Ile    (BCH-408)
```

and the HIV-2 peptide coupled to the solid support is:

```
Lys Val Thr Ala Ile Glu Lys Tyr Leu Gln Asp Gln Ala Arg
                      ┌────────────────────┐
Leu Asn Ser Trp Gly Cys Ala Phe Arg Gln Val Cys His Thr

Thr Val Pro Trp Val Asn Asp Ser            (BCH-202ck).
```

4.    The kit of claim 1, wherein the HTLV-I peptides coupled to the solid support are:

```
Pro His Ser Asn Leu Asp His Ile Leu Glu Pro Ser Ile Pro

Trp Lys Ser Lys Pro Tyr Val Glu Pro Thr Ala Pro Gln Val

Leu                                        (BCH-234);
```

EP 0 605 433 B1

and

```
Ala Ile Val Ser Ser Pro Ser His Asn Ser Leu Ile Leu Pro

Pro Phe Ser Leu Ser Pro Val Pro Thr Leu Gly Ser Arg

                                              (BCH-416)
```

and the HTLV-II peptide coupled to the solid support is:

```
Thr Ser Glu Pro Thr Gln Pro Pro Pro Thr Ser Pro Pro Leu

Leu Val His Asp Ser Asp Leu Glu His Val Leu    (BCH-456).
```

5.  The kit of claim 1, wherein the solid support is a multi-well plastic microtiter plate.

6.  A method of making the immunoassay kit of claim 1, comprising the steps of:
    (a) first coupling the HTLV-I and HTLV-II peptides to the solid support;
    (b) then coupling the HIV-1 and HIV-2 peptides to the solid support.

7.  The method of claim 6, wherein said HTLV-I peptides, said HTLV-II peptides, said HIV-1 peptides and said HIV-2 peptides are first diluted with 0.05M sodium carbonate-bicarbonate buffer to yield respectively an HTLV-I peptide solution, an HTLV-II peptide solution, an HIV-1 peptide solution and an HIV-2 peptide solution before coupling to the solid support.

8.  The method of claim 7, wherein said HTLV-I peptide solution, said HTLV-II peptide solution, said HIV-1 peptide solution and said HIV-2 peptide solution are each at a peptide concentration of between 5 $\mu$g/ml and 10 $\mu$g/ml.

9.  The method of claim 7, wherein said HTLV-I and HTLV-II peptide solutions are mixed to yield an HTLV peptide cocktail before coupling to the solid support.

10. The method of claim 9, wherein said HTLV peptide cocktail has a total peptide concentration of between 5 $\mu$g/ml and 10 $\mu$g/ml.

11. The method of claim 7, wherein said HIV-1 and HIV-2 peptide solutions are mixed to yield an HIV peptide cocktail before coupling to the solid support.

12. The method of claim 11, wherein said HIV peptide cocktail has a total peptide concentration of between 5 $\mu$g/ml and 10 $\mu$g/ml.

13. A method of using the immunoassay kit of claim 1, comprising the steps of:
    (a) applying a biological specimen to the solid support;
    (b) removing the excess biological specimen from the solid support;
    (c) washing the coated solid support;
    (d) treating the washed solid support with an appropriate label;
    (e) assaying for the presence of label on the solid support.

14. The method of claim 13, wherein said biological specimen is chosen from the group consisting of blood, plasma, serum, saliva, urine, tears, milk and cerebral fluid.

15. The method of claim 14, wherein said biological specimen is serum.

16. The method of claim 13, wherein said biological specimen is first diluted with a specimen buffer before applying it to the solid support.

17. The method of claim 13, wherein step (b) is performed using aspiration.

18. The method of claim 13, wherein said label is peroxidase labeled anti-human immunoglobulin G or per-

19

oxidase labeled anti-human immunoglobulin M.

**Patentansprüche**

1. Immunoassay-Kit zur gleichzeitigen Bestimmung von Antikörpern gegen HIV-1, HIV-2, HTLV-I und HTLV-II, der mindestens ein Peptid aus jeder von vier Gruppen von Peptiden, gebunden an einen festen Träger, enthält, wobei die Peptide der vier Gruppen die Formeln a-X-b, a-Y-b, a-Z-b und a-W-b aufweisen, worin

X ausgewählt ist unter

Arg Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser　　　　　　　　　　(BCH-87c),

Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser　　　　　　　　　　(BCH-87ck),

Lys Ile Glu Pro Leu Gly Val Ala Pro Thr Lys Ala Lys Arg

Arg Val Val Gln Arg Glu Lys Arg　　　　　　　　(BCH-132),

Lys Lys Ile Leu Ala Val Glu Arg Tyr Leu Lys Asp Gln Gln

Leu Leu Gly Ile Trp Gly Cys Ser Gly Lys Leu Ile Cys Thr

Thr Ala Val Pro Trp Asn Ala Ser　　　　　　　　(BCH-266),

Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser Gly Lys Leu Ile　　(BCH-408)

und

Lys Lys Ile Leu Ala Val Glu Arg Tyr Leu Lys Asp Gln Gln

Leu Leu Gly Ile Trp Gly Cys Ser Gly Lys Leu Ile Cys Thr

Thr Ala Val Pro Trp Asn Ala Ser Gly Lys Leu Ile

(BCH-408k)

sowie deren Analoga,
Y ausgewählt ist unter

Arg Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser          (BCH-202c),

Lys Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser          (BCH-202ck),

Lys Lys Val Thr Ala Ile Glu Lys Tyr Leu Gln Asp Gln Ala

Arg Leu Asn Ser Trp Gly Cys Ala Phe Arg Gln Val Cys His

Thr Thr Val Pro Trp Val Asn Asp Ser          (BCH-265),

.Lys Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser Ala Phe Arg Gln Val

(BCH-417)

und

Lys Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser Gly Lys His Ile

(BCH-422)

sowie deren Analoga,

Z ausgewählt ist unter

```
Pro His Ser Asn Leu Asp His Ile Leu Glu Pro Ser Ile Pro
Trp Lys Ser Lys Pro Tyr Val Glu Pro Thr Ala Pro Gln Val
Leu                                           (BCH-234)
```

und

```
Ala Ile Val Ser Ser Pro Ser His Asn Ser Leu Ile Leu Pro
Pro Phe Ser Leu Ser Pro Val Pro Thr Leu Gly Ser Arg
                                              (BCH-416)
```

sowie deren Analoga
und
W ausgewählt ist unter

```
Leu Val His Asp Ser Asp Leu Glu His Val Leu Thr Pro Ser
Thr Ser Trp Thr Thr Lys Ile Leu            (BCH-219),
```

```
Thr Ser Glu Pro Thr Gln Pro Pro Pro Thr Ser Pro Pro Leu
Leu Val His Asp Ser Asp Leu Glu His Val Leu    (BCH-456)
```

und

```
Thr Ser Glu Pro Thr Gln Pro Pro Pro Thr Ser Pro Pro Leu
Val His Asp Ser Asp Leu Glu His Val Leu        (BCH-486)
```

sowie deren Analoga,
wobei

a        ein Amino-Terminus, 1 bis 8 Aminosäuren oder ein Substituent ist, der wirksam ist, um die Kupplung des Peptids zu erleichtern oder seine immunogene oder antigene Wirksamkeit zu erhöhen, und

b        ein Carboxy-Terminus, 1 bis 8 Aminosäuren oder ein Substituent ist, der wirksam ist, um die Kupplung des Peptids zu erleichtern oder seine immunogene oder antigene Wirksamkeit zu erhöhen.

2.    Kit nach Anspruch 1, wobei die Peptide durch Festphasen-Peptidsynthese hergestellt sind.

3.    Kit nach Anspruch 1, wobei die an den festen Träger gekuppelten HIV-1-Peptide

```
Lys Ile Glu Pro Leu Gly Val Ala Pro Thr Lys Ala Lys Arg
Arg Val Val Gln Arg Glu Lys Arg            (BCH-132)
```

und

```
Lys Ile Leu Ala Val Glu Arg Tyr Leu Lys Asp Gln Gln Leu

         ┌─────────────────────┐
Leu Gly Ile Trp Gly Cys Ser Gly Lys Leu Ile Cys Thr Thr

Ala Val Pro Trp Asn Ala Ser Gly Lys Leu Ile     (BCH-408)
```

sind
und das an den festen Träger gekuppelte HIV-2-Peptid

```
Lys Val Thr Ala Ile Glu Lys Tyr Leu Gln Asp Gln Ala Arg

         ┌───────────────┐
Leu Asn Ser Trp Gly Cys Ala Phe Arg Gln Val Cys His Thr

Thr Val Pro Trp Val Asn Asp Ser              (BCH-202ck).
```

ist.

4. Kit nach Anspruch 1, wobei die an den festen Träger gekuppelten HTLV-I-Peptide

```
Pro His Ser Asn Leu Asp His Ile Leu Glu Pro Ser Ile Pro

Trp Lys Ser Lys Pro Tyr Val Glu Pro Thr Ala Pro Gln Val

Leu                                          (BCH-234)
```

und

```
Ala Ile Val Ser Ser Pro Ser His Asn Ser Leu Ile Leu Pro

Pro Phe Ser Leu Ser Pro Val Pro Thr Leu Gly Ser Arg

                                             (BCH-416)
```

sind
und das an den festen Träger gekuppelte HTLV-II-Peptid

```
Thr Ser Glu Pro Thr Gln Pro Pro Pro Thr Ser Pro Pro Leu

Leu Val His Asp Ser Asp Leu Glu His Val Leu    (BCH-456)
```

ist.

5. Kit nach Anspruch 1, wobei der feste Träger eine Kunststoff-Mikrotiterplatte mit zahlreichen Vertiefungen ist.

6. Verfahren zur Herstellung des Immunoassay-Kits nach Anspruch 1, das folgende Schritte umfaßt:
   (a) zunächst Kupplung der HTLV-I- und HTLV-II-Peptide an den festen Träger sowie
   (b) anschließende Kupplung der HIV-1- und HIV-2-Peptide an den festen Träger.

7. Verfahren nach Anspruch 6, wobei die HTLV-I-Peptide, die HTLV-II-Peptide, die HIV-1-Peptide und die HIV-2-Peptide zuerst mit 0,05 M Natriumcarbonat-hydrogencarbonat-Puffer unter Erhalt einer HTLV-I-Peptidlösung, einer HTLV-II-Peptidlösung, einer HIV-1-Peptidlösung bzw. einer HIV-2-Peptidlösung verdünnt werden, bevor sie an den festen Träger gekuppelt werden.

**8.** Verfahren nach Anspruch 7, wobei die HTLV-I-Peptidlösung, die HTLV-II-Peptidlösung, die HIV-1-Peptidlösung und die HIV-2-Peptidlösung jeweils eine Peptidkonzentration von 5 bis 10 µg/ml aufweisen.

**9.** Verfahren nach Anspruch 7, wobei die HTLV-I-Peptidlösung und die HTLV-II-Peptidlösung zu einer HTLV-Peptid-Mischlösung gemischt werden, bevor die Kupplung an den festen Träger vorgenommen wird.

**10.** Verfahren nach Anspruch 9, wobei die HTLV-Peptid-Mischlösung eine Gesamt-Peptidkonzentration von 5 bis 10 µg/ml aufweist.

**11.** Verfahren nach Anspruch 7, wobei die HIV-1-Peptidlösung und die HIV-2-Peptidlösung zu einer HIV-Peptid-Mischlösung gemischt werden, bevor die Kupplung an den festen Träger vorgenommen wird.

**12.** Verfahren nach Anspruch 11, wobei die HIV-Peptid-Mischlösung eine Gesamt-Peptidkonzentration von 5 bis 10 µg/ml aufweist.

**13.** Verwendung des Immunoassay-Kits nach Anspruch 1, die folgende Schritte umfaßt:
(a) Aufbringen einer biologischen Probe auf den festen Träger;
(b) Entfernung von überschüssiger biologischer Probe vom festen Träger;
(c) Waschen des beschichteten festen Trägers;
(d) Behandeln des gewaschenen festen Trägers mit einem entsprechenden Markierungsmittel und
(e) Testen auf Vorliegen des Markierungsmittels am festen Träger.

**14.** Verfahren nach Anspruch 13, wobei die biologische Probe unter Blut, Plasma, Serum, Speichel, Urin, Tränenflüssigkeit, Milch und Cerebralflüssigkeit ausgewählt ist.

**15.** Verfahren nach Anspruch 14, wobei die biologische Probe Serum ist.

**16.** Verfahren nach Anspruch 13, wobei die biologische Probe zuerst mit einem Probenpuffer verdünnt wird, bevor sie auf den festen Träger aufgebracht wird.

**17.** Verfahren nach Anspruch 13, wobei Schritt (b) durch Absaugen durchgeführt wird.

**18.** Verfahren nach Anspruch 13, wobei das Markierungsmittel mit Peroxidase markiertes anti-Humanimmunglobulin G oder mit Peroxidase markiertes anti-Humanimmunglobulin M ist.

## Revendications

**1.** Trousse d'immunodosage pour détecter simultanément des anticorps contre HIV-1, HIV-2, HTLV-I et HTLV-II, comprenant au moins un peptide de chaque groupe parmi quatre groupes de peptides, couplé à un support solide, les groupes de peptides ayant les formules a-X-b, a-Y-b, a-Z-b et a-W-b, où:
X est choisi dans le groupe consistant en:

Arg Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser                    (BCH-87c)

Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser                    (BCH-87ck)

Lys Ile Glu Pro Leu Gly Val Ala Pro Thr Lys Ala Lys Arg

Arg Val Val Gln Arg Glu Lys Arg                (BCH-132)

Lys Lys Ile Leu Ala Val Glu Arg Tyr Leu Lys Asp Gln Gln

Leu Leu Gly Ile Trp Gly Cys Ser Gly Lys Leu Ile Cys Thr

Thr Ala Val Pro Trp Asn Ala Ser                (BCH-266)

Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser Gly Lys Leu Ile    (BCH-408)

Lys Lys Ile Leu Ala Val Glu Arg Tyr Leu Lys Asp Gln Gln

Leu Leu Gly Ile Trp Gly Cys Ser Gly Lys Leu Ile Cys Thr

Thr Ala Val Pro Trp Asn Ala Ser Gly Lys Leu Ile

                                               (BCH-408k)

et leurs analogues;

Y est choisi dans le groupe consistant en :

Arg Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser                (BCH-202c)

Lys Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser    (BCH-202ck)

Lys Lys Val Thr Ala Ile Glu Lys Tyr Leu Gln Asp Gln Ala

Arg Leu Asn Ser Trp Gly Cys Ala Phe Arg Gln Val Cys His

Thr Thr Val Pro Trp Val Asn Asp Ser   (BCH-265)

Lys Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser Ala Phe Arg Gln Val

           (BCH-417)

Lys Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser Gly Lys His Ile

           (BCH-422)

et leurs analogues ;

Z est choisi dans le groupe consistant en :

Pro His Ser Asn Leu Asp His Ile Leu Glu Pro Ser Ile Pro

Trp Lys Ser Lys Pro Tyr Val Glu Pro Thr Ala Pro Gln Val

Leu           (BCH-234)

Ala Ile Val Ser Ser Pro Ser His Asn Ser Leu Ile Leu Pro

Pro Phe Ser Leu Ser Pro Val Pro Thr Leu Gly Ser Arg

           (BCH-416)

et leurs analogues ;

W est choisi dans le groupe consistant en :

EP 0 605 433 B1

```
Leu Val His Asp Ser Asp Leu Glu His Val Leu Thr Pro Ser

Thr Ser Trp Thr Thr Lys Ile Leu                    (BCH-219)
```

```
Thr Ser Glu Pro Thr Gln Pro Pro Pro Thr Ser Pro Pro Leu

Leu Val His Asp Ser Asp Leu Glu His Val Leu        (BCH-456)
```

```
Thr Ser Glu Pro Thr Gln Pro Pro Pro Thr Ser Pro Pro Leu

Val His Asp Ser Asp Leu Glu His Val Leu            (BCH-486)
```

et leurs analogues ;

a est une extrémité amino de 1 à 8 acides aminés ou un substituant efficace pour faciliter le couplage ou pour améliorer l'activité immunogène ou antigénique du peptide ; et

b est une extrémité carboxy de 1 à 8 acides aminés ou un substituant efficace pour faciliter le couplage ou pour améliorer l'activité immunogène ou antigénique du peptide.

2.	Trousse selon la revendication 1, dans laquelle les peptides sont formés par synthèse en phase solide.

3.	Trousse selon la revendication 1, dans laquelle les peptides de HIV-1 couplés au support solide sont :

```
Lys Ile Glu Pro Leu Gly Val Ala Pro Thr Lys Ala Lys Arg

Arg Val Val Gln Arg Glu Lys Arg                    (BCH-132);
```

et

```
Lys Ile Leu Ala Val Glu Arg Tyr Leu Lys Asp Gln Gln Leu

Leu Gly Ile Trp Gly Cys Ser Gly Lys Leu Ile Cys Thr Thr

Ala Val Pro Trp Asn Ala Ser Gly Lys Leu Ile       (BCH-408)
```

et le peptide de HIV-2 couplé au support solide est :

```
Lys Val Thr Ala Ile Glu Lys Tyr Leu Gln Asp Gln Ala Arg

Leu Asn Ser Trp Gly Cys Ala Phe Arg Gln Val Cys His Thr

Thr Val Pro Trp Val Asn Asp Ser                    (BCH-202ck).
```

4.	Trousse selon la revendication 1, dans laquelle les peptides de HTLV-I couplés au support solide sont :

```
Pro His Ser Asn Leu Asp His Ile Leu Glu Pro Ser Ile Pro

Trp Lys Ser Lys Pro Tyr Val Glu Pro Thr Ala Pro Gln Val

Leu                                                (BCH-234);
```

et

27

```
Ala Ile Val Ser Ser Pro Ser His Asn Ser Leu Ile Leu Pro

Pro Phe Ser Leu Ser Pro Val Pro Thr Leu Gly Ser Arg

                                              (BCH-416)
```

et le peptide de HTLV-II couplé au support solide est :

```
Thr Ser Glu Pro Thr Gln Pro Pro Pro Thr Ser Pro Pro Leu

Leu Val His Asp Ser Asp Leu Glu His Val Leu      (BCH-456).
```

5.  Trousse selon la revendication 1, dans laquelle le support solide est une microplaque de titrage en plastique à puits multiples.

6.  Procédé de fabrication de la trousse d'immunodosage de la revendication 1, comprenant les étapes suivantes :
    (a) tout d'abord couplage des peptides de HTLV-I et de HTLV-II au support solide ;
    (b) puis couplage des peptides de HIV-1 et de HIV-2 au support solide.

7.  Procédé selon la revendication 6, dans lequel lesdits peptides de HTLV-I, lesdits peptides de HTLV-II, lesdits peptides de HIV-1 et lesdits peptides de HIV-2 sont tout d'abord dilués avec du tampon carbonate-bicarbonate de sodium 0,05 M pour donner respectivement une solution de peptides de HYLV-I, une solution de peptides de HTLV-II, une solution de peptides de HIV-1 et une solution de peptides de HIV-2 avant le couplage au support solide.

8.  Procédé selon la revendication 7, dans lequel ladite solution de peptides de HTLV-I, ladite solution de peptides de HTLV-II, ladite solution de peptides de HIV-1 et ladite solution de peptides de HIV-2 sont chacune à une concentration en peptides comprise entre 5 µg/ml et 10 µg/ml.

9.  Procédé selon la revendication 7, dans lequel lesdites solutions de peptides de HTLV-I et HTLV-II sont mélangées pour donner une combinaison de peptides de HTLV avant le couplage au support solide.

10. Procédé selon la revendication 9, dans lequel ladite combinaison de peptides de HTLV a une concentration totale en peptides comprise entre 5 µg/ml et 10 µg/ml.

11. Procédé selon la revendication 7, dans lequel lesdites solutions de peptides de HIV-1 et HIV-2 sont mélangées pour donner une combinaison de peptides de HIV avant le couplage au support solide.

12. Procédé selon la revendication 11, dans lequel ladite combinaison de peptides de HIV a une concentration totale en peptides comprise entre 5 µg/ml et 10 µg/ml.

13. Procédé d'utilisation de la trousse d'immunodosage de la revendication 1, comprenant les étapes consistant à :
    (a) appliquer un échantillon biologique au support solide ;
    (b) retirer du support solide l'échantillon biologique en excès ;
    (c) laver le support solide recouvert ;
    (d) traiter le support solide lavé avec un marqueur approprié ;
    (e) tester la présence de marqueur sur le support solide.

14. Procédé selon la revendication 13, dans lequel ledit échantillon biologique est choisi dans le groupe consistant en le sang, le plasma, le sérum, la salive, l'urine, les larmes, le lait et le liquide cérébral.

15. Procédé selon la revendication 14, dans lequel ledit échantillon biologique est le sérum.

16. Procédé selon la revendication 13, dans lequel ledit échantillon biologique est tout d'abord dilué avec un tampon d'échantillon avant d'être appliqué sur le support solide.

17. Procédé selon la revendication 13, dans lequel l'étape (b) est accomplie par aspiration.

18. Procédé selon la revendication 13, dans lequel ledit marqueur est un anti-immunoglobine G humaine marqué à la peroxydase ou un anti-immunoglobine M humaine marqué à la peroxydase.